(19) Europäisches Patentamt / European Patent Office / Office européen des brevets

(11) **EP 2 434 952 B1**

(12) **EUROPEAN PATENT SPECIFICATION**

(45) Date of publication and mention
of the grant of the patent:
**11.01.2017 Bulletin 2017/02**

(21) Application number: **10728297.2**

(22) Date of filing: **25.05.2010**

(51) Int Cl.:
**A61B 5/1455** $^{(2006.01)}$

(86) International application number:
**PCT/IB2010/052306**

(87) International publication number:
**WO 2010/136962 (02.12.2010 Gazette 2010/48)**

(54) **APPARATUS AND METHOD FOR SPECTROPHOTOMETRIC MEASUREMENTS OF BLOOD PARAMETERS**

VORRICHTUNG UND VERFAHREN FÜR SPEKTROFOTOMETRISCHE MESSUNGEN VON BLUTPARAMETERN

APPAREIL ET PROCÉDÉ DE MESURES SPECTROPHOTOMÉTRIQUES DE PARAMÈTRES SANGUINS

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **26.05.2009 IT MI20090926**

(43) Date of publication of application:
**04.04.2012 Bulletin 2012/14**

(73) Proprietor: **B. Braun Avitum AG**
**34212 Melsungen (DE)**

(72) Inventors:
• **PORRO, Giampiero**
  **22100 Como (IT)**

• **POZZI, Roberto**
  **20142 Milano (IT)**
• **TORINESI, Alessandro**
  **20049 Concorezzo (MI) (IT)**

(74) Representative: **Winter, Brandl, Fürniss, Hübner, Röss, Kaiser, Polte - Partnerschaft mbB**
**Patent- und Rechtsanwaltskanzlei**
**Alois-Steinecker-Strasse 22**
**85354 Freising (DE)**

(56) References cited:
**WO-A1-94/27495      WO-A1-2008/136548**
**US-A- 5 615 672      US-A- 6 144 444**
**US-A1- 2006 189 926      US-A1- 2009 043 171**
**US-B1- 6 208 880**

**Description**

The present invention has as its object an apparatus and a method for spectrophotometry measurements of blood parameters.

The field of application of the present invention is the medical field and in particular the monitoring of blood parameters during the course of therapies that require extracorporeal blood circulation like for example: haemodialysis, plasmapheresis, extracorporeal membrane oxygenation (ECMO), conservation of transplant organs and regional oncological therapies.

Hereafter we shall refer to haemodialysis treatment as the preferred field of application, but it should be understood that the applicability of the present invention is not exclusive to this field.

Haemodialysis is a replacement therapy to kidney function, which is applied to subjects that have little or no kidney activity (renal failure).

In extracorporeal haemodialysis blood is withdrawn by needles or similar directly from an artery and, through a join surgically created between a vein and an artery, it is treated and returned to the patient through an external circuit. The flow inside the circuit is fed through a peristaltic pump. The external circuit comprises a dialyzer, i.e. an element with a double compartment in which a semipermeable membrane of suitably porosity is used. In a first compartment the blood is made to flow, and in a second compartment an aqueous solution (dialyzing solution), enriched with the solutes that it is necessary to give to the blood and low in or without those to be taken out, is made to flow.

During dialysis treatment it is necessary to continuously control the blood parameters like for example the hematocrit and the oxygen saturation.

In particular, the hematocrit, the ratio between the corpuscular part and the liquid aliquot of the blood, is the indicator of the amount of liquid present in the blood.

These and other properties, as known, can be measured through spectrophotometry, i.e. through the study of the electromagnetic spectra of visible light, of near ultraviolet and near infrared.

In particular, these techniques make use of an emitting device and a detecting device of electromagnetic waves so that the detector of electromagnetic waves is able to measure the amount of electromagnetic energy absorbed or reflected by the blood.

Based on the known electromagnetic properties of blood, which vary according to its composition, it is possible to determine the parameters of interest.

The first applications of this technique to the measurement of the blood parameters in vitro by taking blood from the patient and laboratory analysis, are described in the edition Science of 22 June 1951; 113(2974):723-5.

In more recent times, there have been many patents dealing with the measurement of the hematocrit and oxygen saturation of blood carried out in extracorporeal circuits; in particular we quote utility patents US 6,090,061 and US 7,381,195.

The utility patent US 6,090,061 describes a particular type of cuvette, able to be used in an extracorporeal circuit for blood, suitable for the use of spectrophotometry.

The cuvette is a container provided with an inlet duct and an outlet duct to be connected to the duct of the extracorporeal blood. It is also provided with two parallel and opposite flat surfaces where the electromagnetic emitter and detector are positioned, facing the bloodflow and one another. In order that the measurement can be carried out with a high degree of precision, the space between emitter and receiver must be small, so that the light emitted by the emitter can be detected by the detector. However, it is disadvantageous to narrow the duct, due to the loss of energy that would be given to the bloodflow. For this purpose, some protuberances are provided, on one of the two parallel surfaces described above, projecting towards the inside of the duct, which allow an accurate measurement without creating a throttling of the duct.

However, the aforementioned solution is not without drawbacks, like for example the impossibility of being able to install said system on any type of extracorporeal blood duct, without providing an interruption of the duct and thus a duct in two parts.

The utility patent US 7,381,195 B2 describes a hematocrit sensor that partially solves the problems of the previous solution, by foreseeing the use of sensors applied to the blood duct, without interruptions of the duct or narrowing.

The sensors are positioned along the duct in contiguous positions arranged on a line parallel to the axis of the tubing.

The aforementioned sensors are an emitting device and a detecting device of electromagnetic radiation, positioned so that they are in optical connection with the bloodflow, and so that the detecting device can receive the light reflected by the blood.

The device foresees the use of a carcass containing the emitting/receiving devices, inside of which the blood duct passes. On the separating surface between the emitting and receiving devices and the blood duct some openings are formed, shaped like elongated slits or circular. Said openings have adjustable dimensions.

The operation of the device comprises an emission step and a reception step of the light reflected by the blood.

The light that is detected by the receiver is translated into a potential difference and processed by a processing unit. The length of the slit is adjusted so as to make the detected signal proportional to the hematocrit value over time.

The system in this case is unable to proceed to a measurement of the hematocrit or of the other parameters of interest in an absolute manner, i.e. it restores variations of the value of the parameter with respect to a first measurement. With this method a first compensation of background noise, i.e. the light that enters the device from the outside, is possible.

A further parameter that it would be advisable to detect is the presence of gaseous emboli inside the bloodflow to be returned to the patient.

The detection of gaseous emboli is of fundamental importance in systems used for parenteral nutrition, infusion of drugs, blood transfusion and extracorporeal circulation since they can cause even irreversible damage to the patient being treated.

One of the causes of the entry of gaseous emboli into the blood is joints that are not perfectly airtight applied to the ducts.

The techniques for measuring gaseous emboli can be of two types: optical or ultrasound type.

The optical technique of the state of the art is based on the difference in the amount of light transmitted between the air and the water media (the blood and the infusion substances mostly consist of water).

In particular, a measurement of the variation in electromagnetic intensity caused by the absorption and by the diffusion by the blood or by the infusion substance is carried out. Usually, the analysis of blood through analogue circuits does not allow optimal filtering of the signal.

Patent US 6,529,751 describes the use of electromagnetic sources in the field from 800 nm to 850 nm to quantify microemboli in the blood.

However, at these wavelengths it is not possible to compensate the variations in optical transmissivity due to the variations in hematocrit, the value of which can vary even by 300% during the infusion of drugs.

Related prior art is disclosed in US 6,144,444. Therein an apparatus for monitoring blood parameters during cardio-pulmonary bypass surgery or during other procedures which utilize an extracorporeal circuit, is disclosed. The apparatus is typically used to monitor the percentage of haemoglobin bound with oxygen (oxygen saturation), the total amount of haemoglobin in the blood, and the percentage of blood which is comprised of red blood cells (hematocrit), although the apparatus can be adapted to measure other blood parameters. The apparatus (or monitor) provides real time results to show immediate changes (trending) in the monitored parameters.

Related prior art is also disclosed in US 2009/0043171 A1. Therein systems and methods for determining a physiological parameter in a patient are provided. In certain embodiments, a system can include an analyte detection system configured to measure first analyte data in a fluid sample received from a patient, a medical sensor configured to measure second analyte data in the patient, and a processor configured to receive the first analyte data and the second analyted data and to determine a physiological parameter based at least in part on the first analyte data and the second analyte data. In certain such embodiments, the medical sensor may be a pulse oximeter, and the physiological parameter may include a cardiovascular parameter including, for example, cardiac output.

A disposable cuvette through which passes pulsatile flowing blood is disclosed in WO 94/27495 A1. The cuvette has a conduit with two opposed walls having a predetermined separation therebetween that varies with each pulse of the flowing blood. A sealed air pocket damps the variation of the predetermined separation. The conduit is comprised of materials which permit passage therethrough of wavelengths of selected electromagnetic radiation. The radiation is emitted from a photoemitter which, and after passing through the cuvette, is detected by a photodetector. The quantities of detected radiation are operated on by a computer which uses a spectrophotometry technique to derive therefrom a blood constituent concentration value. Preferably, both the cuvette and the spectrophotometry technique are used during hemodialysis to derive changes in the hematocrit value of the blood of a dialyzed patient, thereby to deduce therefrom changes in the blood volume of the patient during dialysis.

WO 2008/136548 discloses a device for measuring and monitoring hemoglobin values through a blood tube, comprising

- a groove for fitting the blood tube;
- two emitting units to be directly in contact with the fitted blood tube;
- first light receiving unit for receiving reflected light;
- second light receiving unit for receiving transmitted light;
- CPU for controlling the probe and processing input data;
- a sliding lid for covering and fixing the fitted blood tube.

The purpose of the present invention is therefore to overcome the drawbacks of the prior art.

A first task of the present invention is to measure some blood parameters of interest, for wide operating conditions and with high precision. In particular, the parameters of interest considered are: hematocrit, oxygen saturation, blood temperature and the presence of gaseous microemboli.

The range of variation of some of these parameters depends varies from patient to patient and generally:

- hematocrit comprised between 10% and 55%;

- oxygen saturation between 40% and 100%;

- blood temperature between 10°C and 45°C;

- hematic flows comprised between 10 ml/min and 8000 ml/min.

In order to obtain a useful and significant measurement, the imprecision of these measurements must be preferably no more than ± 5 percentage units for the hematocrit value and the oxygen saturation value and ± 0,5°C for the temperature value.

A second task of the present invention is to carry out the measurements directly on the tubing without it having particular optical and/or geometric characteristics.

A third task of the present invention is to be able to carry out an absolute measurement of the hematocrit value and oxygen saturation.

A further task of the present invention is to provide an apparatus capable of detecting the presence in the blood of gaseous emboli, and in particular their number and their size.

The purpose and the tasks indicated above are achieved by an apparatus and method in accordance with what is claimed in claim 1 and in claim 8, respectively.

The further advantages and the characteristics of the present invention will become clear from the following detailed description of some example embodiments, made for indicating and not limiting purposes, in relation to the attached drawings, in which:

Fig. 1 shows a perspective view from the front of the measurement apparatus according to the invention, with the cover raised;

Fig. 2 shows a perspective view of emitting and detecting means according to the invention;

Fig. 3 shows a plan view of the emitting and detecting means according to the invention;

Fig. 4 shows a longitudinal section view according to the section plane FV - FV of fig. 3 of the measurement apparatus according to the invention;

Fig. 5 shows a perspective view from the front of the measurement apparatus according to the invention, without the cover;

Fig. 6 shows a perspective view from the front of the measurement apparatus according to the invention, without the cover with the blood duct inserted;

Fig. 7 shows a perspective view of a cross section of the measurement apparatus according to the invention, seen from the front, with the blood duct inserted and with the cover in operating position;

Fig. 8 shows a block diagram of the apparatus according to the invention;

Fig. 9 shows a graph of the pulses emitted to carry out the measurement according to the invention;

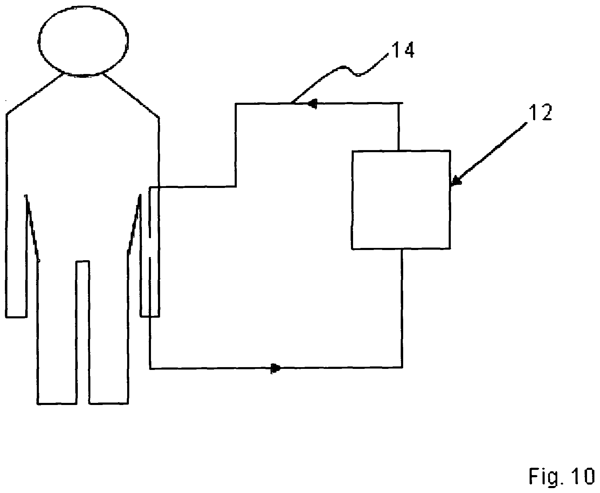

Fig. 10 shows a diagram of the operative positioning of the apparatus according to the invention;

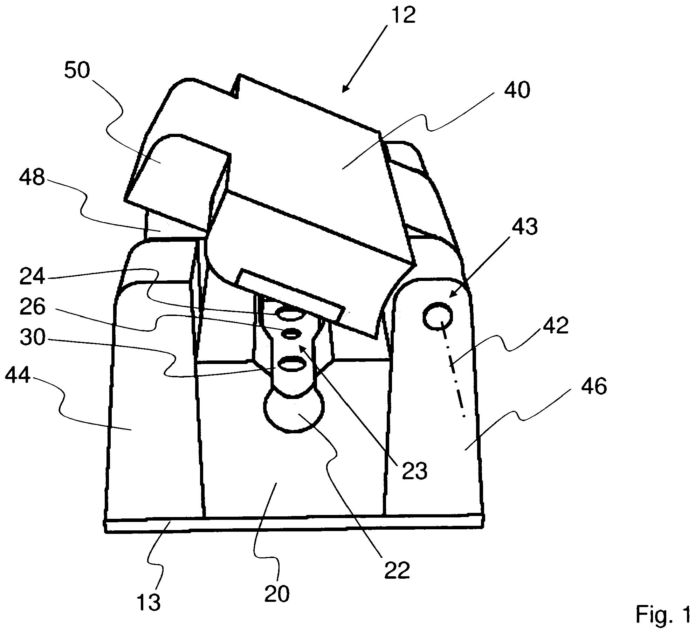

Fig. 1 1 shows a graph of the signals used for detecting and measuring gaseous emboli, in particular:

(a) emitted signal;

(b) detected signal;

(c) sampled signal;

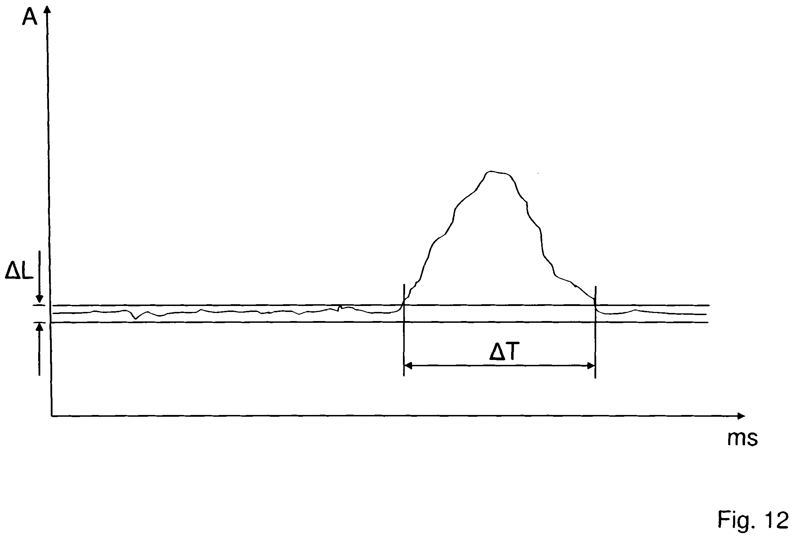

Fig. 12 shows a graph of the signal processed for detecting and measuring gaseous emboli;

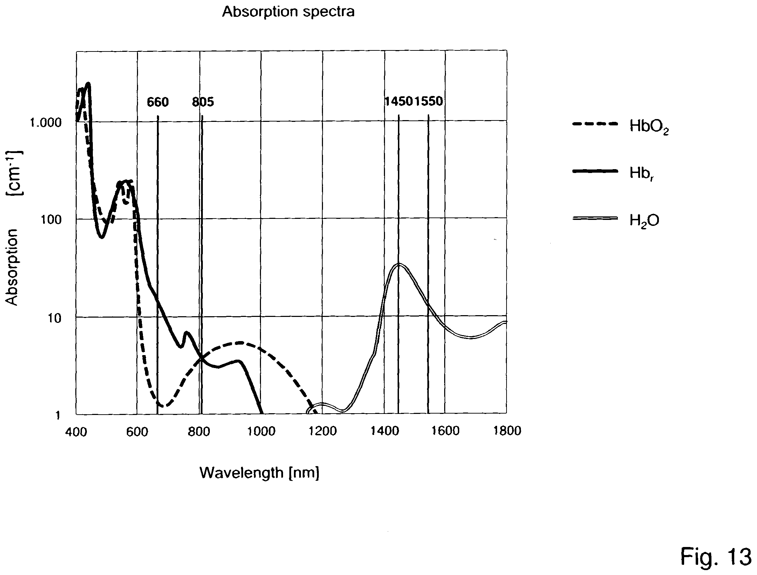

Fig. 13 shows a graph relating to the absorption spectra of water and of haemoglobin in oxidised and reduced form.

Figures 1,4,6 and 8 represent an apparatus for measuring the blood parameters in an extracorporeal circuit according to the invention, wholly indicated with reference numeral 12.

The apparatus 12 according to the invention comprises a seat 23 suitable for containing a duct 14 for the blood flow in said extracorporeal circuit.

Electromagnetic radiation emitting means 16 and electromagnetic radiation detecting means 18 face the seat 23. Said means 16, 18 are also connected to a control unit 38.

The apparatus 12 according to the invention is characterised in that said emitting means 16 are suitable for producing electromagnetic radiation at different wavelengths and the detecting means 18 are suitable for detecting the electromagnetic radiation diffused in the blood at said wavelengths. Moreover, the apparatus 12 is characterised in that the control unit 38 is suitable for calculating values of blood parameters through a correlation between reference values and ratios obtained from values of the light intensity of the radiation detected at at least two different wavelengths.

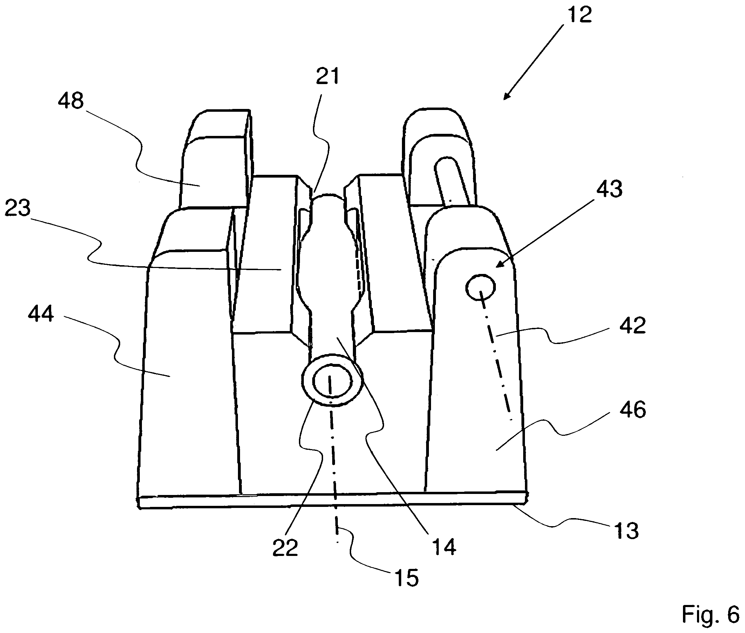

Referring to the apparatus 12, shown in fig. 6 with the duct 14 inside the seat 23, the following are unequivocally determined: an axial direction parallel to an axis 15 of the duct 14 when inserted in the seat 23; a radial direction perpendicular to the axial direction and passing through the axis 15 of the duct 14; and a transversal plane containing the radial direction and perpendicular to the axial direction.

The apparatus 12 according to a first embodiment of the invention comprises a body 20 suitable for containing inside it the means 16 and 18 connected to a base 13.

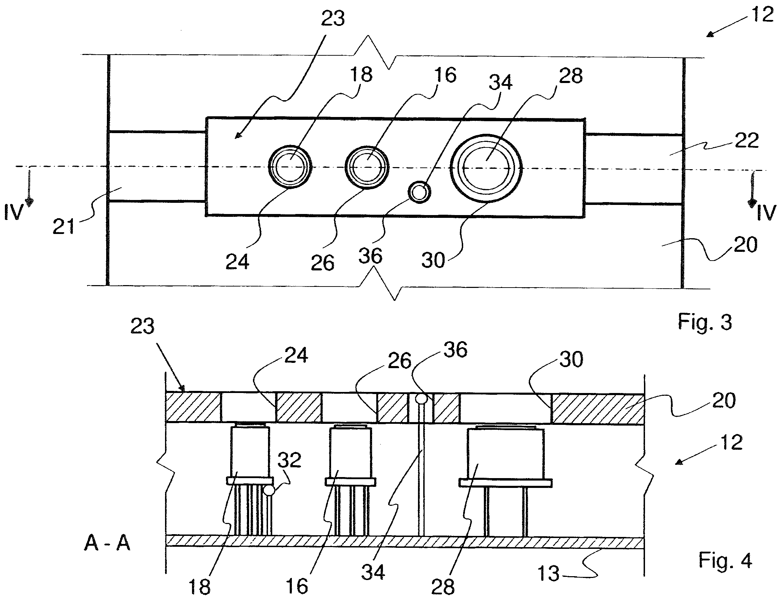

The body 20 is associated with the base 13 so that the means 16 and 18 are contained inside the space located between body 20 and base 13 as shown in fig. 4.

On the face opposite the base 13 of the body 20, a seat 23 is arranged, comprising three parts having a different section in the transversal plane:

- two end seats 21 and 22 substantially countershaped with respect to the duct 14, which extend in the transversal plane for about two-thirds of the circumference of the duct 14.
- a central seat 23 with substantially rectangular section in the transversal plane, at which the means 16, 18 are housed.

In correspondence of the central seat 23 holes 24 and 26 are formed, arranged in a substantially radial direction, for the optical connection between the duct 14 and the detecting means 18 and emitting means 16, respectively.

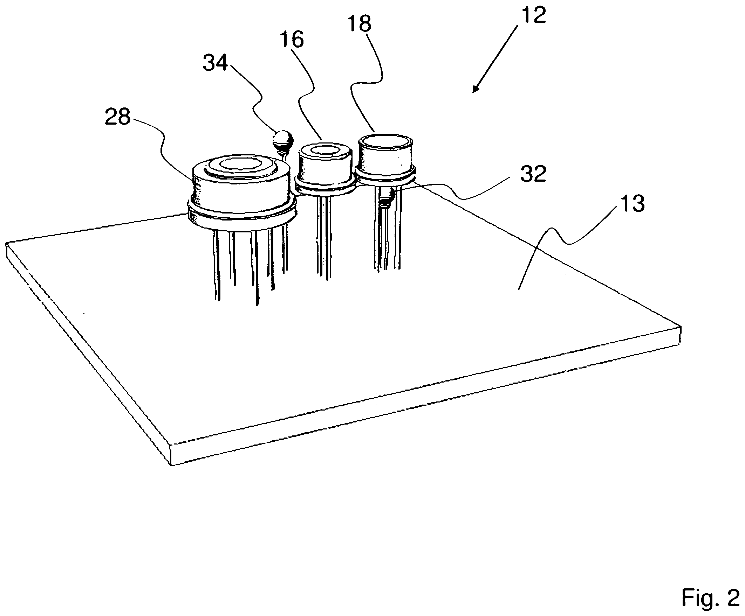

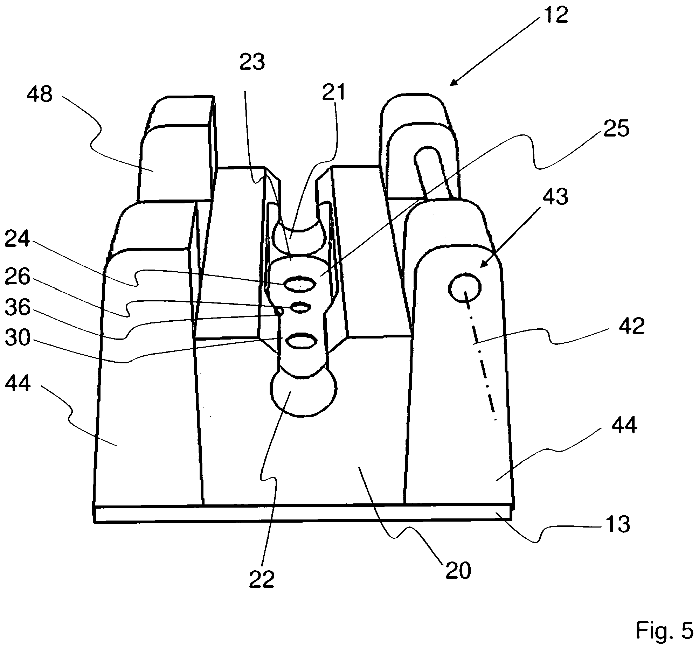

As can be seen, in the embodiments of figures 1 to 5, the emitting means 16 and the detecting means 18, axially spaced, are arranged on the same side of the seat 23. In accordance with such a configuration, the measurement carried out by the apparatus 12 takes place in reflection, since the detecting means 18 measure the fraction of light reflected by the blood.

A first shoulder 44 and a second shoulder 46 are rigidly fixed on two parallel sides of the body. The apparatus also comprises a cover 40 able to rotate around an axis 42 positioned on a side of the cover 40 itself and parallel to a side of the body 20. In particular, the rotation axis is formed by means of a hinge 43 that connects the cover 40 and the second shoulder 46.

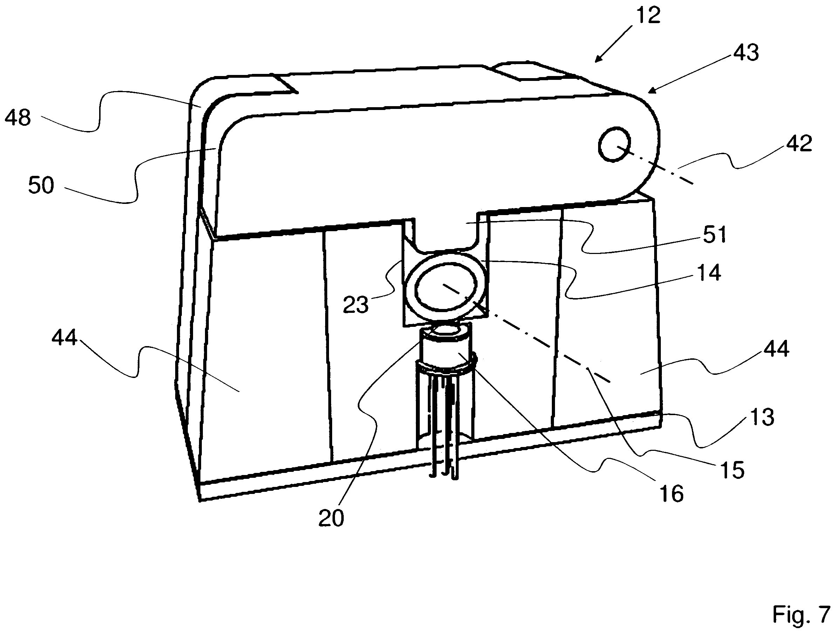

The cover 40 has a projection 51 (as shown in fig. 7) such as to couple with the seat 23 for the duct 14, so as to cover the portion of circumference of the duct 14 not covered by the body 20 .

On the first shoulder 44 a recess 48 is formed for a coupling surface 50 formed on the cover 40.

When the duct 14 for the blood is inserted into the body 20 and the cover 40 is closed, the duct 14 is slightly squashed by the projection 51 thus creating two parallel flat surfaces: one in contact with the cover 40 and one in contact with the body 20 at the holes 24, 26 for the means 16, 18.

The slight squashing effect visible in fig. 7 has two purposes:

- blocking the mutual position between apparatus 12 and duct 14;
- creating a substantially flat surface in which the measurement can take place.

The duct 14, selected among one of those commonly used in the medical field, is substantially transparent to the wavelengths of the emitted radiation. For example, the duct 14 consists of a polymer like plasticized PVC. Moreover, if the duct 14 is hit by electromagnetic radiation, the part of said radiation absorbed by the duct 14 must be less than 50% for each individual used wavelength of the total emitted radiation.

In other words, the duct 14 which is introduced into the seat 23 and through which the blood parameters are measured, is any section of a common disposable extracorporeal circuit. Thus it is not necessary, for the purposes of the interaction with the apparatus 12 according to the invention, to provide a section of duct with particular characteristics, either from a geometric point of view, or from the point of view of the optical properties of the walls.

The electromagnetic radiation emitting means 16 comprise light emitting diodes (LED) (not shown).

In particular, in accordance with an embodiment of the invention, the emitting means 16 comprise a single LED for each of the wavelengths that are used for calculating the blood parameters. As well known to the man skilled in the art, the characteristics of some particular types of LED involve an emission spectrum that assumes the profile of a very narrow bell, with a very pronounced peak at a particular wavelength. Such LEDs, considered hereafter, have a very narrow emission spectrum and have no secondary emissions. For this reason, the approximation is commonly accepted based on which each type of LED is attributed with one wavelength only.

The emitting means 16 preferably comprise at least two different LEDs, each dedicated to the emission of electromagnetic radiation at one single wavelength.

In accordance with an embodiment of the invention, the emitting means 16 comprise four different LEDs: a first LED (A) suitable for emitting light with a wavelength equal to 805nm, a second LED (B) suitable for emitting light with a wavelength equal to 660nm, a third LED (C) suitable for emitting light with a wavelength equal to 1450nm and finally a fourth LED (D) suitable for emitting light with a wavelength equal to 1550nm.

The electromagnetic radiation detecting means 18 comprise a wide band sensor for example of the InGaAs type. The InGaAs sensor is a semiconductor made up of indium, gallium and arsenic which is typically sensitive to the band of electromagnetic radiation within the range from 600 nm up to 2600 nm.

According to an embodiment of the invention it is intended to use a temperature detector 28 measuring the bloodflow temperature, placed inside the apparatus in a position adjacent to the emitting means 16 and also connected to the base 13.

In particular, the temperature detector 28 has a wide band reception within the range of middle-infrared electromagnetic radiation up to 15000 nm.

The temperature detector 28 measures the bloodflow temperature and, in the case in which it is an infrared detector, it is in light communication with the duct 14 through a hole 30 adjacent to the hole 26.

The temperature detector 28 makes it possible to take also into account, in the calculation of the blood parameters, the influence of the blood temperature itself. Indeed, keeping every other condition the same, the optical characteristics of blood vary as the temperature varies, i.e. the proportions between the amount of radiation absorbed and diffused are altered. Such a variation is monotonic with respect to the values of the electromagnetic radiation used and therefore to a large extent the measurement error is compensated by adopting a rateometric measurement technique as described hereafter. Such a variation of the optical characteristics of blood can be determined experimentally for each electromagnetic radiation adopted, hi particular, it is possible to observe a variation for amounts of absorbed and diffused electromagnetic radiation that on average is about 0.25% for a temperature variation of 1°C. Therefore, it is possible to take into account such a variation by providing the control unit 38 with the signal of the temperature detector 28.

According to an embodiment of the invention it is foreseen to use a first temperature sensor 32 measuring the operating temperature of the detector 18 arranged in contact with it, and connected to the base 13.

The use of the first temperature sensor 32 is justified by the fact that the responsivity of the detection device 18 depends upon the temperature, which can translate into a drift of the measured hematocrit value by about 0.5% for a temperature variation of 1 °C. The drift can be seen through a comparison between the data obtained by measuring a blood sample through spectrophotometry and the value obtained from the same blood sample from laboratory apparatuses commonly used in the medical field (for example a centrifuge).

According to an embodiment it is also foreseen to use a second temperature sensor 34, which controls the operation of the temperature detector 28.

The sensor 34 is placed in contact with the duct 14 through the hole 36 formed between the hole 26 and the hole 30 and measures the blood temperature through contact with the duct 14.

The second sensor 34 is used as a safety sensor in the case of malfunction of the temperature detector 28.

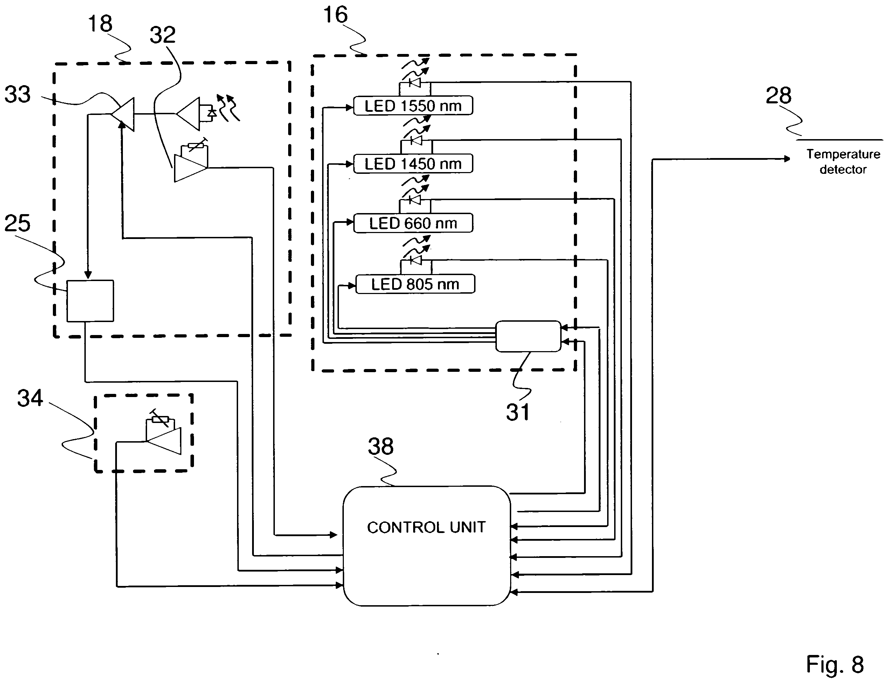

Now referring to the block diagram of Fig. 8, the emitting means 16 and the detecting means 18 are connected to a control unit 38. The control unit 38 regulates the power supply currents for the emitting device 16, i.e. of the light emitting diodes, through a digital to analogue converter with resolution preferably of no less than 12 bit.

The guide current of each element is generated and regulated through the combination of a field effect transistor (FET) and an operational amplifier connected to a digital to analogue converter with resolution preferably of no less than 12 bit indicated with reference numeral 31.

The detecting means 18 convert the radiation diffused into current and then into a voltage through a transimpedance amplifier. A second amplification is digitally controlled through a variable analogue gain amplifier 33, the output voltage of which is converted into a digital signal through an analogue to digital converter with resolution preferably of at least 16 bit, indicated with reference numeral 25.

The temperature sensor 32 is connected to the control unit 38 described earlier that, according to calibration tables of the receiving instrument, takes care of correcting the signal acquired by the detecting means 18.

In Fig. 8 it is also possible to see that the control unit 38 has the temperature detector 28 connected to it.

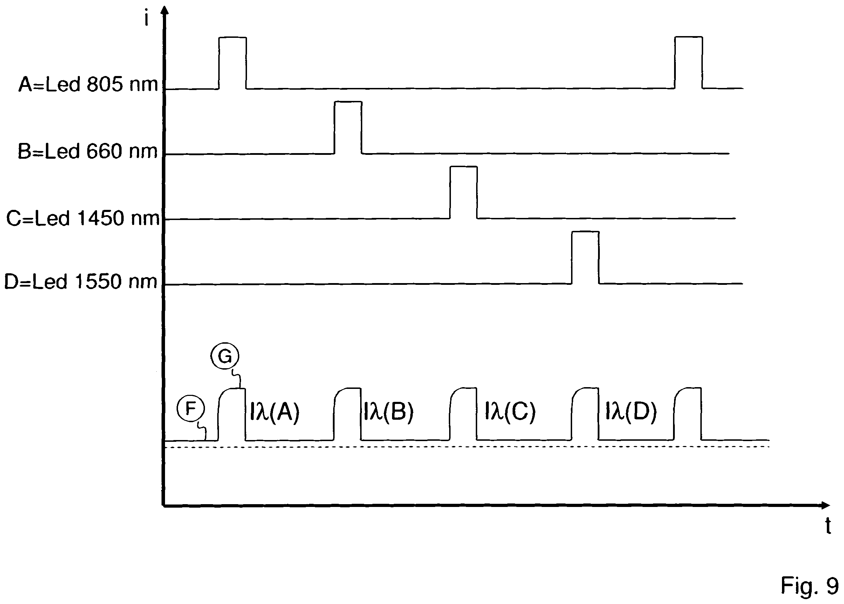

We shall now describe in detail the operation of the apparatus for measuring blood parameters just described, also referring to figure 9.

From the functional point of view it can be intuitively appreciated that the radiation emitted by the emitting means 16 strikes the duct 14 and in part is attenuated and in part diffused in the blood.

The detecting means 18 detect the electromagnetic radiation diffused or emitted by the blood which is a function of the concentration of the biologic constituents and of its temperature.

As described above, the LEDs of the emitting means 16 are suitable for emitting single pulses of radiation, each of which has a selected and very precise wavelength. Such a characteristic of the apparatus 12 thus makes it possible to use extremely simple detecting means 18. Such a characteristic, in particular, makes it superfluous to use a spectrophotometer that analyses the radiation reflected by the blood. It should be considered in particular that the spectrophotometer, used in the prior art, comprises a prism and a plurality of detecting elements arranged so as to create a practically continuous detection band to receive the spectrum generated by the prism. The greater the number of such detecting elements, the better the approximation of the continuum that is obtained. In a common spectrophotometer used for analyses similar to those that are the purpose of the present invention, 128, 256 or more of such detecting elements can be used. In light of the structure described above, the man skilled in the art can easily understand how the spectrophotometer is a rather delicate (for example for the optical alignment of the components) and rather expensive (for example for the plurality of detecting elements used, that have sensitivity to electromagnetic radiation also in the spectral field of the near-infrared) component.

The detecting means 18 used in the apparatus 12 according to the invention can, in principle, comprise a single detecting element compared to the 128, 256 or even more used in the prior art. Therefore, there is no need to analyse the wavelength of the detected radiation since it is already selected at source thanks to the use of LEDs in the emitting means 16.

The wavelengths of the electromagnetic radiation emitted are within the range from 660 nm to 1550 nm, whereas those diffused or emitted by the blood and able to be detected by the measuring apparatus are within the range from 600 nm to 15000 nm.

Preferably, there are at least two wavelengths emitted by the emitting means 16, selected at the water absorption peaks and at particular transitions of the absorption spectra of $HbO_2$ and $Hb$ molecules.

In particular, to calculate a single blood parameter, for example the hematocrit (Ht%) or the oxygen saturation ($sO_2$%), there are at least two wavelengths emitted since, with the measurement of the ratio between the intensity detected at two different wavelengths correlated to reference values, it is possible to minimise the influence upon the measurement of all those parameters that in varying can alter the precision. Such parameters are, for example, the drift of the electrical and optical characteristics of the sources and of the photodetectors caused by ageing, by thermal variations and contributions of electromagnetic radiation that are not diffused directly by the blood.

Said reference values are values obtained through the normal analysis techniques. The reference values can also be presented in the form of a calibration curve.

In practice, a calibration of the apparatus is carried out by correlating the value of the ratios between different wavelengths with values calculated in the laboratory through the usual techniques, e.g. for the case of the hematocrit the calculation is carried out through centrifuging of the blood in microcapillaries.

For the calculation of two blood parameters, for example hematocrit (Ht%) and oxygen saturation ($sO_2$%) at least three different wavelengths are needed since at least two ratios are necessary between intensities detected at different wavelengths to be correlated to reference values.

According to an embodiment of the invention described hereafter four different wavelengths and therefore three ratios between the signal received at said wavelengths are used.

In this way it is possible to calculate a value of one of the parameters (Ht% or $sO_2$%) so that it can be used to calculate the other one.

It is thus possible to take into account the dependency of Ht% upon $sO_2$% or of $sO_2$% upon Ht%.

Preferably, the four wavelengths emitted by the emitting means 16 are 805nm (A), 660nm (B), 1450nm (C) and 1550nm (D). Such wavelengths were selected according to the following criteria:

-    805nm corresponds to the isosbestic absorption point of haemoglobin in oxygenated form ($HbO_2$) and in reduced form (Hb);
-    660nm corresponds to the point at which the maximum absorption difference of $HbO_2$ with respect to Hb is recorded; and
-    1450nm and 1550nm correspond to a high water absorption area.

Regarding this, let us consider the graph of figure 13. As can be seen, the absorption values at the indicated frequencies are extremely significant. As the man skilled in the art can easily understand, the fact that it is chosen to analyse the wavelengths at high absorptions (for water) and at characteristic values (for haemoglobin) helps to isolate the phenomenon of interest from other factors (typically from the background noise or ambient light).

As can be clearly seen in figure 13, the water absorption peak at the wavelength of 1450 nm, and the area at 1550 nm near to it, are particularly important for determining the hematocrit. Indeed, it is possible to make the ratio between the absorption of the blood at the wavelengths of 1450 nm and 1550 nm, due therefore substantially to water, and that at 805 nm, where on the other hand there is no absorption by water.

Similarly, the choice of the wavelength of 660 nm where the absorption difference between the oxygenated form of haemoglobin and the non-oxygenated or reduced form is at its maximum, makes it possible to measure the percentage oxygen saturation of the blood by making a ratio with the absorption at 805 nm where the two forms of haemoglobin have the same absorption.

The emitting means 16 emit a sequence of pulses at different wavelengths, for example the sequence can be the one represented in fig. 9, i.e. A-B-C-D (805nm, 660nm, 1450nm, 1550nm).

The frequency at which the pulses are emitted is constant, but their intensity is variable according to their wavelength.

This choice has been made to compensate for the different absorption in the blood and to optimise, in the field of variation of the parameters to be measured, the dynamics of the electromagnetic radiation signal detected for each wavelength. The variation in intensity is determined for each LED by the control unit 38.

The diffused radiation is detected by the detecting means 18 in a synchronous manner but it is slightly delayed with respect to the emitted radiation, so as to wait for the stable level condition.

The intensities of the detected radiations are therefore $I_\lambda(A)$, $I_\lambda(B)$, $I_\lambda(C)$ and $I_\lambda(D)$.

They can then be converted by the detecting means 18 and optimised in level, for each wavelength, through the variable gain amplifier 33.

Of course, the intensity of the detected electromagnetic radiation will be the sum of two contributions: a first contribution due to the radiation diffused by the blood and a second contribution due to a background noise, i.e. light radiation detected when the light source is switched off.

As can be seen from figure 9, the background noise corresponds to the value of the electromagnetic radiation measured at point F, whereas the total value, diffused by the blood and due to the light emitting diode, measured at the pulse is that at point G.

The actual value of the amount of diffused radiation is obtained as the difference between the value corresponding to point G and the value corresponding to point F. For a correct removal of the effect due to the background radiation, it is important for the measurement of the background radiation (point F) to be carried out within 10 $\mu$s from the start of the pilot pulse of the light emitting diode.

In order to measure the hematocrit value Ht% according to the invention, the ratio $R_1 = I_\lambda(A)/[I_\lambda(C)+I_\lambda(D)]$ is calculated using the intensities of electromagnetic radiation diffused by the blood and detected at three different wavelengths. Such a ratio is correlated to the values obtained through laboratory measurements through the use of "gold standard" apparatuses. The result of such a correlation is a mathematical function of the third order that links the measurement of $R_1$ to the values of Ht:

$$\text{Ht\%} = [R_1^3 * \alpha_3 + R_1^2 * \alpha_2 + R_1 * \alpha_1 + \alpha_0] + a * (SO_2\% - 75) \text{ where } \alpha_0, \alpha_1, \alpha_2, \alpha_3 \text{ and } \alpha$$

where $\alpha_0$, $\alpha_1$, $\alpha_2$, $\alpha_3$ and $\alpha$ are experimentally obtained coefficients.

In order to calculate $sO_2\%$ a further intensity of detected radiation generated by a further wavelength is used, and preferably the ratio $R_2 = I_\lambda(A)/I_\lambda(B)$ is used. Thus by using the signal generated and detected at two different wavelengths such a ratio is correlated to the values obtained through laboratory measurements through the use of "gold standard" apparatuses. The result of such correlation is a mathematical function of the second order that links the measurement of $R_2$ to the values of $sO_2$ % given here:

$$sO_2\% = [R_2^2 * \alpha_1 (\text{Ht\%}) + R_2 * \alpha_2 (\text{Ht\%}) + \alpha_0 (\text{Ht\%})]$$

where the coefficients $\alpha_1(\text{Ht\%})$, $\alpha_2(\text{Ht\%})$ and $\alpha_0$ (Ht%) are experimentally obtained as a function of the value of Ht%, considering the variation range divided into three fields, the first for values of Ht%<25, the second field for Ht%>40 and the third intermediate field for values of Ht% comprised between 25 and 40.

According to an embodiment of the invention, the value of $sO_2\%$ is corrected with a function of the value of Ht% calculated previously. In particular, therefore, the reference values for $sO_2\%$ will be a function of the value of Ht%, and thus of $R_1$ and of $R_2$.

In accordance with an embodiment of the invention the value of Ht% is corrected through a function of the value

of $sO_2$%.

Both the hematocrit value, and the oxygen saturation value thus calculated can be corrected based on the responsivity of the detecting means 18, through the sensor 32, and based on the blood temperature through the temperature detector 28.

In other words, the hematocrit value is correlated to calibration values measured in the laboratory and takes into account the responsivity of the detecting means 18, the temperature variations of the blood, and the variation of oxygen saturation. In the same way, the oxygen saturation value is correlated to calibration values measured in the laboratory and takes into account the responsivity of the detecting means 18, the temperature variations of the blood, and the variation of the hematocrit value of the blood.

The measurement of the hematocrit and oxygen saturation values can with this method also be carried out by using just three wavelengths, in an analogous manner to what has just been seen.

Now referring to figures 1 1 and 12 we shall describe in detail the method for detecting gaseous emboli according to the invention.

The detection of gaseous emboli is obtained through three main steps.

A first step consists of emitting a train of pulses of constant intensity at a determined frequency. The effects of the measurement are not affected by the used wavelength, but preferably a wavelength is used that has high optical efficiency as a function of the used detecting means.

Referring to the apparatus 12 described earlier it is thus possible to use, for example, one of the four wavelengths A, B, C or D.

For example, it is possible to use the wavelength C = 1450nm.

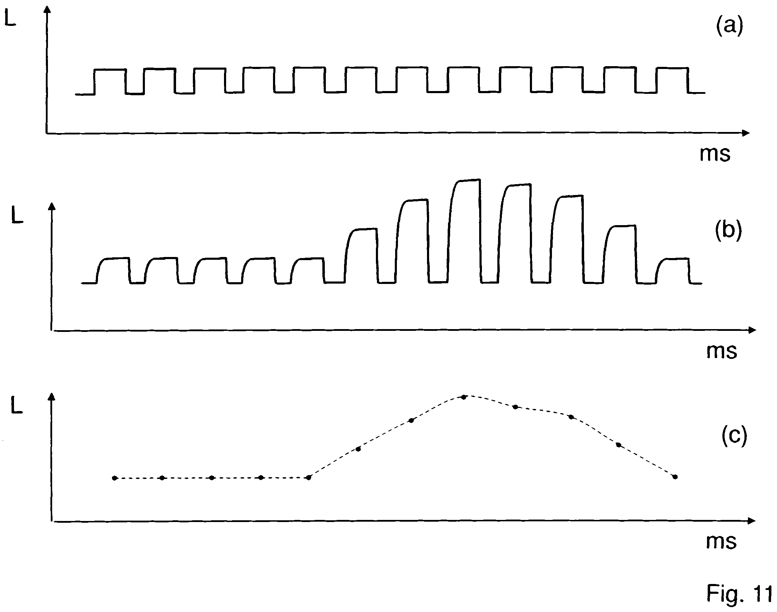

In a possible embodiment of the method in a first step indicated in figure 11 (a) the emitting means 16 emit a signal at the wavelength C and at the frequency preferably of no less than 13.33KHz within the time period in which the emitting device 16 does not emit the wavelength C used to measure hematocrit or oxygen saturation.

The second step, referring to figure 11 (b) is the detection of the electromagnetic radiation diffused by the blood at said wavelength.

The detected radiation has the same characteristics as the radiation used to calculate the hematocrit or oxygen saturation value.

The third step consists of processing the signal detected in the previous step.

The value of the width L of the signal is used for sampling to be used to extrapolate the curve indicated in figure 11 (c) and 12.

Said curve describes the trend of the intensity of the detected electromagnetic radiation and therefore the variations in trend undergone by the curve that, when above a certain threshold, are interpreted as the presence of emboli.

Referring to fig. 12, the apparatus according to the invention also allows a link between size of the embolus and variation of the curve.

In particular, ∆L indicates a tolerance range within which the detected radiation can vary without causing alarms due to the presence of emboli.

As soon as the intensity of the detected signal exceeds the threshold, a step known as "START" begins in which the measurement of a time starts.

When the signal goes back within the tolerance there is an "END" step in which the time measurement is interrupted.

The time passed between "START" and "END" is known as ∆T and it is used to calculate the size of the embolus.

In particular, the diameter of the embolus if assimilated to an air bubble is given by:

$$d = \Delta T \cdot v$$

d: diameter of the air bubble;

∆T: "END" time - "START" time;

v: velocity of the fluid that can be obtained through the value of the flow rate and of the section of the duct 14.

It is thus clear how the parameters that influence the measurement are:

- value of the tolerance range;
- frequency of emission of the train of pulses; and
- fluid velocity.

According to an embodiment of the invention it is possible to carry out a computation of the volume of air accumulated both as a single embolus and as a sum of microemboli and consequently generate an alarm and/or a stop of the bloodflow if a risk threshold determined for the patient is exceeded.

A possible embodiment of the present invention can foresee the use of a plurality of emitting means and a plurality of detecting means, differently positioned with respect to the axial direction.

The architecture of the apparatus has been described as an example of an embodiment, and it is thus possible to foresee other embodiments, for example by foreseeing a cover that can be rigidly fixed to the rest of the structure through the use of screws or similar.

In a possible embodiment it is possible to foresee further safety sensors that can interact with those already present.


**Claims**

1. Apparatus (12) for measuring the blood parameters in an extracorporeal circuit, comprising:

   a seat (23) suitable for containing a duct (14) for the blood flow of said extracorporeal circuit;
   electromagnetic radiation emitting means (16);
   electromagnetic radiation detecting means (18), said means (16, 18) facing the seat (23);
   a first temperature sensor (32) for measuring the operating temperature of the detecting means (18);
   a second temperature sensor (34) suitable for measuring the blood temperature,
   a central body (20) suitable for containing inside it the means (16) and (18) connected to a base (13) and
   a control unit (38) to which said emitting means (16) and said detecting means (18) are connected;
   wherein
   the electromagnetic radiation emitting means (16) and electromagnetic radiation detecting means (18) are contained inside the space between the body (20) and the base (13), holes (24, 26) are formed on the seat for optical connection between the duct (14) and the detecting means (18) and emitting means (16),
   said electromagnetic radiation emitting means (16) are suitable for producing electromagnetic radiation at different wavelengths, said electromagnetic radiation detecting means (18) are suitable for detecting the electromagnetic radiation diffused in the blood at said wavelengths, and in that the control unit (38) is suitable for calculating values of blood parameters through a correlation between reference values and ratios obtained from values of the light intensity of the radiation detected at at least two different wavelengths,
   wherein the duct (14) is any section of a disposable extracorporeal circuit wherein the values of the blood parameters calculated are corrected based on the blood temperature and/or are corrected based on the value of the operating temperature of the detecting means (18) and
   wherein the apparatus comprises a cover (40), said cover (40) able to rotate around an axis (42), being parallel to a side of the body (20),
   said cover having a projection (51), which is adapted to squash the duct (14), inserted into the seat (23) in the body (20), when the cover (40) is closed,
   wherein two parallel flat surfaces are created in the duct in a way, that one surface is in contact with the cover (40) and the other surface is in contact with the body (20) at the holes (24, 26) for the radiation emitting means (16) and radiation detecting means (18).

2. Apparatus (12) according to claim 1 **characterised in that** said emitting means (16) are suitable for emitting electromagnetic radiation at four different wavelengths and said emitting means (16) are preferably suitable for emitting electromagnetic radiation at wavelengths: A=805nm, B=660nm, C=1450nm, D=1550nm.

3. Apparatus (12) according to any one of the preceding claims **characterised in that** said emitting means (16) are suitable for emitting electromagnetic radiation at wavelengths corresponding to the water absorption peaks and/or it comprises a temperature detector (28) facing the seat (23) and connected to the control unit (38), wherein the temperature detector (28) is preferably an infrared temperature sensor having a wide band reception in the range of middle-infrared electromagnetic radiation up to 15000 nm.

4. Apparatus (12) according to any one of the preceding claims **characterised in that** said emitting means (16) emit a train of impulses of constant width and frequency at at least one wavelength.

5. Apparatus (12) according to any one of the preceding claims **characterised in that** it comprises:

the central body (20) comprising the seat (23);
the base (13), associated with the central body (20), to which the emitting and detecting means (16, 18) are connected so that they are contained inside the space located between body (20) and base (13); and
said holes (24, 26), formed on the seat (23) are distributed in a substantially axial direction.

6. Apparatus (12) according to any one of the preceding claims **characterised in that** it comprises additional holes (30, 36) formed on the seat (23) and distributed in a substantially axial direction, for the optical connection between the duct (14) and the temperature detector (28) and for the contact between the second sensor (34) and the duct (14), respectively.

7. Method for measuring blood parameters using the apparatus of claim 1, said method comprising the steps of:

inserting a duct for blood into the seat in the body,
closing the cover of the body,
squashing the duct with the
protrusion on the cover, thereby forming two parallel flat
surfaces,
emitting electromagnetic radiation through the parallel flat surfaces in the form of a sequence of pulses of at least two different wavelenghts;
detecting the radiation diffused in the blood by said at least two wavelengths;
calculating the values of the blood parameters correlating reference values to the ratio between the intensity of the electromagnetic radiation detected at said at least two different wavelengths.

8. Method according to the previous claim **characterised in that** the oxygen saturation value ($sO_2\%$) is obtained by correlating to calibration curves the ratio $R_2 = I_\lambda(A)I_\lambda(B)$ between a first intensity of detected electromagnetic radiation $I_\lambda(A)$ and a second intensity of detected electromagnetic radiation $I_\lambda(B)$ and/or by correlating the ratio $R_2 = I_\lambda(A)/I_\lambda(B)$ between intensity of detected electromagnetic radiation to calibration values approximated with a mathematical function of the second order.

9. Method according to any one of claims 7 or 8 **characterised in that** the hematocrit value (Ht%) is obtained by correlating to calibration curves the ratio $R_1 = I_\lambda(A)/[I_\lambda(C)+I_\lambda(D)]$ between the intensities of electromagnetic radiation deteced $I_\lambda(A)$, $I_\lambda(C)$ and $I_\lambda(D)$ and/or by correlating the ratio $R_1 = I_\lambda(A)/[I_\lambda(C)+I_\lambda(D)]$ between the intensities of electromagnetic radiation detected to calibration values approximated with a mathematical function of the third order.

10. Method according to one of claims 7 to 9 **characterised in that** the calculated oxygen saturation value of the blood ($sO_2\%$ is corrected based on the hematocrit value (Ht%).

11. Method according to one of claims 7 to 10 **characterised in that** the calculated hematocrit value (Ht%) of the blood is corrected based on the oxygen saturation value ($SO_2\%$).

12. Method according to any one of claims 7 to 11 comprising the steps of:

emitting a train of pulses at constant intensity and frequency;
detecting the train of pulses; and
processing the detected train of pulses and
associating the presence and size of an embolus with the time period in which the variation in intensity of the detected radiation exceeds a certain threshold.

13. Method according to any of the claims 7 to 13 **characterised in that** a computation of the volume of air accumulated both as single embolus and as a sum of microemboli is carried out and consequently an alarm and/or a stop of the blood flow is generated if a risk threshold determined for the patient is exceeded.

**Patentansprüche**

1. Vorrichtung (12) zum Messen der Blutparameter in einem extrakorporalen Kreislauf, aufweisend:

einen Sitz (23), der geeignet ist, einen Kanal (14) für die Blutströmung des extrakorporalen Kreislaufs aufzunehmen;

eine elektromagnetische Strahlung aussendende Einrichtung (16);

eine elektromagnetische Strahlung erfassende Einrichtung (18), wobei die Einrichtungen (16, 18) dem Sitz (23) zugewandt ist;

einen ersten Temperatursensor (32) zum Messen der Betriebstemperatur der erfassenden Einrichtung (18);

einen zweiten Temperatursensor (34), der zum Messen der Bluttemperatur geeignet ist,

einen zentralen Körper (20), der geeignet ist, darin die mit einer Basis (13) verbundenen Einrichtungen (16) und (18) aufzunehmen, und

eine Steuereinheit (38), mit der die emittierende Einrichtung (16) und die erfassende Einrichtung (18) verbunden sind; wobei

die elektromagnetische Strahlung aussendende Einrichtung (16) und die elektromagnetische Strahlung erfassende Einrichtung (18) innerhalb des Raumes zwischen dem Körper (20) und der Basis (13) untergebracht sind, Löcher (24, 26) für eine optische Verbindung zwischen dem Kanal (14) und der erfassenden Einrichtung (18) und emittierenden Einrichtung (16) auf dem Sitz ausgebildet sind, die elektromagnetische Strahlung aussendende Einrichtung (16) ist dazu geeignet elektromagnetische Strahlung mit verschiedenen Wellenlängen zu erzeugen, die elektromagnetische Strahlung erfassende Einrichtung (18) ist dazu geeignet elektromagnetische Strahlung, die im Blut bei den Wellenlängen gestreut wird, zu detektieren, und die Steuereinheit (38) ist dazu geeignet, die Werte von Blutparametern zu kalkulieren, durch eine Korrelation zwischen Referenzwerten und Verhältnissen, die aus Werten der Lichtintensität der detektierten Strahlung bei mindestens zwei verschiedenen Wellenlängen erhalten werden,

wobei der Kanal (14) irgendein Abschnitt eines wegwerfbaren extrakorporalen Kreislaufs ist, wobei die Werte der berechneten Blutparameter basierend auf der Bluttemperatur und/oder basierend auf dem Wert der Betriebstemperatur der erfassenden Einrichtung (18) korrigiert werden und

wobei die Vorrichtung eine Abdeckung (40) aufweist, wobei die Abdeckung (40) fähig ist, um eine Achse (42) zu rotieren, die parallel zu einer Seite des Körpers (20) ist, die Abdeckung hat einen Vorsprung (51), welcher angepasst ist den in den Sitz (23) im Körper (20) eingesetzten Kanal (14) zusammenzudrücken, wenn die Abdeckung geschlossen wird,

wobei zwei parallele, flache Flächen in dem Kanal in einer Weise ausgebildet werden, dass eine Fläche mit der Abdeckung (40) in Kontakt ist und die andere Fläche mit dem Körper (20) an den Löchern (24, 26) für die Strahlung emittierende Einrichtung (16) und die Strahlung erfassende Einrichtung (18) in Kontakt ist.

2. Vorrichtung (12) nach Anspruch 1, **dadurch gekennzeichnet, dass** die emittierende Einrichtung (16) geeignet ist, elektromagnetische Strahlung mit vier verschiedenen Wellenlängen zu emittieren und die emittierende Einrichtung (16) vorzugsweise geeignet ist, elektromagnetische Strahlung zu emittieren mit den Wellenlängen: A = 805nm, B = 660nm, C = 1450nm, D =1550 nm.

3. Vorrichtung (12) nach einem der vorhergehenden Ansprüche, **dadurch gekennzeichnet, dass** die emittierende Einrichtung (16) geeignet ist, elektromagnetische Strahlung bei Wellenlängen zu emittieren, die den Wasserabsorptionspeaks entsprechen und/oder einen Temperaturdetektor (28) aufweist, der dem Sitz (23) zugewandt ist und mit der Steuereinheit (38) verbunden ist, wobei der Temperaturdetektor (28) vorzugsweise ein Infrarot-Temperatursensor ist mit einem Breitbandempfang im Bereich von mittlerer-Infrarot elektromagnetischer Strahlung bis zu 15000nm.

4. Vorrichtung (12) nach einem der vorhergehenden Ansprüche, **dadurch gekennzeichnet, dass** die emittierende Einrichtung (16) eine Folge von Pulsen konstanter Breite und Frequenz bei mindestens einer Wellenlänge emittiert.

5. Vorrichtung (12) nach einem der vorhergehenden Ansprüche, **dadurch gekennzeichnet, dass** sie aufweist:

den zentralen Körper (20), der den Sitz (23) aufweist;

die dem zentralen Körper (20) zugeordnete Basis (13), mit der die emittierende und erfassende Einrichtung (16, 18) so verbunden sind, dass sie innerhalb des zwischen dem Körper (20) und der Basis (13) angeordneten Raums enthalten sind; und

die an dem Sitz (23) ausgebildeten Löcher (24, 26), die in einer im Wesentlichen axialen Richtung verteilt sind.

6. Vorrichtung (12) nach einem der vorhergehenden Ansprüche, **dadurch gekennzeichnet, dass** sie auf dem Sitz (23) ausgebildete und in im Wesentlichen axialer Richtung verteilte zusätzliche Löcher (30, 36), jeweils für die optische Verbindung zwischen dem Kanal (14) und dem Temperaturdetektor (28) und für den Kontakt zwischen dem zweiten Sensor (34) und dem Kanal (14), aufweist.

**7.** Verfahren zum Messen von Blutparametern unter Verwendung der Vorrichtung von Anspruch 1, wobei das Verfahren folgende Schritte aufweist:

Einführen eines Kanals für Blut in den Sitz in dem Körper,
Schließen der Abdeckung des Körpers,
Zusammendrücken des Kanals mit dem Vorsprung auf dem Deckel, wodurch zwei parallele, flache Oberflächen gebildet werden,
Emittieren elektromagnetischer Strahlung durch die parallelen flachen Oberflächen in Form einer Folge von Pulsen von mindestens zwei verschiedenen Wellenlängen;
Erfassen der gestreuten Strahlung im Blut, durch die mindestens zwei Wellenlängen;
Berechnen der Werte der Blutparameter korrelierenden Referenzwerte mit dem Verhältnis zwischen der Intensität der erfassten elektromagnetischen Strahlung, bei den mindestens zwei verschiedenen Wellenlängen.

**8.** Verfahren nach dem vorhergehenden Anspruch, **dadurch gekennzeichnet, dass** der Sauerstoffsättigungswert ($sO_2\%$) erhalten wird durch Korrelation des Verhältnisses $R_2 = I_\lambda(A)I_\lambda(B)$ zwischen einer ersten Intensität von erfasster elektromagnetischer Strahlung $I_\lambda(A)$ und einer zweiten Intensität von erfasster elektromagnetischer Strahlung $I_\lambda(B)$ mit Kalibrationskurven und/oder durch Korrelation des Verhältnisses $R_2 = I_\lambda(A)I_\lambda(B)$ zwischen der Intensität von erfasster elektromagnetischer Strahlung mit Kalibrationswerten angenähert mit einer mathematischen Funktion zweiter Ordnung.

**9.** Verfahren nach einem der Ansprüche 7 oder 8, **dadurch gekennzeichnet, dass** der Hämatokritwert (Ht%) erhalten wird durch Korrelation des Verhältnisses $R_1 = I_\lambda(A)/[I_\lambda(C) + I_\lambda(D)]$ zwischen den Intensitäten von erfasster elektromagnetischer Strahlung $I_\lambda(A), I_\lambda(C)$ und $I_\lambda(D)$ mit Kalibrationskurven und/oder durch Korrelation des Verhältnisses $R_1 = I_\lambda(A)/[I_\lambda(C) + I_\lambda(D)]$ zwischen den Intensitäten von erfasster elektromagnetischer Strahlung mit Kalibrationswerten angenähert mit einer mathematischen Funktion dritter Ordnung.

**10.** Verfahren nach einem der Ansprüche 7 bis 9, **dadurch gekennzeichnet, dass** der berechnete Sauerstoffsättigungswert des Blutes ($sO_2\%$) basierend auf dem Hämatokritwert (Ht%) korrigiert wird.

**11.** Verfahren nach einem der Ansprüche 7 bis 10, **dadurch gekennzeichnet, dass** der berechnete Hämatokritwert des Blutes (Ht%) basierend auf dem Sauerstoffsättigungswert ($sO_2\%$) korrigiert wird.

**12.** Verfahren nach einem der Ansprüche 7 bis 11, aufweisend die Schritte:

Emittieren einer Folge von Pulsen bei konstanter Intensität und Frequenz;
Erfassen der Folge von Pulsen; und
Verarbeiten der erfassten Folge von Pulsen und

Assoziieren der Anwesenheit und Größe eines Embolus mit dem Zeitraum, in dem die Änderung der Intensität der erfassten Strahlung einen bestimmten Schwellenwert überschreitet.

**13.** Verfahren nach einem der Ansprüche 7 bis 13, **dadurch gekennzeichnet, dass** eine Berechnung des Luftvolumens das als einzelner Embolus und als Summe von Mikroemboli akkumuliert wird, durchgeführt wird und somit ein Alarm und/oder ein Stopp des Blutflusses erfolgt, wenn ein Risiko-Schwellwert für den Patienten überschritten wird.

**Revendications**

**1.** Appareil (12) de mesure des paramètres sanguins dans un circuit extracorporel, comprenant :

un logement (23) approprié pour contenir un canal (14) pour l'écoulement de sang dudit circuit extracorporel ;
des moyens d'émission de rayonnement électromagnétique (16) ;
des moyens de détection de rayonnement électromagnétique (18), lesdits moyens (16, 18) faisant face au logement (23) ;
un premier capteur de température (32) pour mesurer la température de fonctionnement des moyens de détection (18) ;
un deuxième capteur de température (34) approprié pour mesurer la température du sang,
un corps central (20) approprié pour contenir à l'intérieur de celui-ci les moyens (16) et (18) raccordés à une base (13)

et

une unité de commande (38) à laquelle lesdits moyens d'émission (16) et lesdits moyens de détection (18) sont raccordés ;

dans lequel

les moyens d'émission de rayonnement électromagnétique (16) et les moyens de détection de rayonnement électromagnétique (18) sont contenus à l'intérieur de l'espace entre le corps (20) et la base, des trous (24, 26) sont formés sur le logement pour un raccordement optique entre le canal (14) et les moyens de détection (18) et les moyens d'émission (16),

lesdits moyens d'émission de rayonnement électromagnétique (16) sont appropriés pour produire un rayonnement électromagnétique à différentes longueurs d'ondes,

lesdits moyens de détection de rayonnement électromagnétiques (18) sont appropriés pour détecter le rayonnement électromagnétique diffusé dans le sang audites longueurs

d'ondes, et en ce que l'unité de commande (38) est appropriée pour calculer des valeurs des paramètres sanguins par une corrélation entre des valeurs de référence et des rapports obtenus à partir des valeurs de l'intensité de lumière du rayonnement détecté à au moins deux longueurs d'ondes différentes,

dans lequel le canal (14) est toute section d'un circuit extracorporel jetable dans lequel les valeurs des paramètres sanguins calculés sont corrigées sur la base de la température du sang et/ou sont corrigées sur la base de la valeur de la température de fonctionnement des moyens de détection (18) et

dans lequel l'appareil comprend un couvercle (40), ledit couvercle (40) pouvant tourner autour d'un axe (42) qui est parallèle à un côté du corps (20),

ledit couvercle ayant une projection (51) qui est adaptée pour écraser le canal (14),

inséré dans le logement (23) dans le corps (20), lorsque le couvercle (40) est fermé,

dans lequel deux surfaces planes parallèles sont créées dans le canal d'une façon telle qu'une surface soit en contact avec le couvercle (40) et l'autre surface soit en contact avec le corps (20) sur les trous (24, 26) pour les moyens d'émission de rayonnement et les moyens de détection de rayonnement (18).

2.   Appareil (12) selon la revendication 1, **caractérisé en ce que** lesdits moyens d'émission (16) sont appropriés pour émettre un rayonnement électromagnétique à quatre longueurs d'ondes différentes et lesdits moyens d'émission (16) sont de préférence appropriés pour émettre un rayonnement électromagnétique à des longueurs d'ondes : A = 805 nm, B = 660 nm, C = 1450 nm, D = 1550 nm.

3.   Appareil (12) selon l'une quelconque des revendications précédentes, **caractérisé en ce que** lesdits moyens d'émission (16) sont appropriés pour émettre un rayonnement électromagnétique à des longueurs d'ondes correspondant aux pics d'absorption d'eau et/ou il comprend un détecteur de température (28) faisant face au logement (23) et raccordé à l'unité de commande (38), dans lequel le détecteur de température (28) est de préférence un détecteur de température à infrarouge ayant une réception à bande large dans la plage de rayonnement électromagnétique infrarouge moyen jusqu'à 15000 nm.

4.   Appareil (12) selon l'une quelconque des revendications précédentes, **caractérisé en ce que** lesdits moyens d'émission (16) émettent un train d'impulsions de largeur et de fréquence constantes à au moins une longueur d'ondes.

5.   Appareil (12) selon l'une quelconque des revendications précédentes, **caractérisé en ce qu'**il comprend :

le corps central (20) comprenant le logement (23),

la base (13), associée au corps central (20), auquel les moyens d'émission et de détection (16, 18) sont raccordés de sorte qu'ils soient contenus à l'intérieur de l'espace situé entre le corps (20) et la base (13) ; et

lesdits trous (24, 26) formés sur le logement (23) sont distribués dans une direction sensiblement axiale.

6.   Appareil (12) selon l'une quelconque des revendications précédentes, **caractérisé en ce qu'**il comprend des trous supplémentaires (30, 36) formés sur le logement (23) et distribués dans une direction sensiblement axiale, pour le raccordement optique entre le canal (14) et le détecteur de température (28) et pour le contact entre le deuxième capteur (34) et le canal (14), respectivement.

7.   Procédé de mesure de paramètres sanguins utilisant l'appareil selon la revendication 1, ledit procédé comprenant les étapes suivantes :

insertion d'un canal pour le sang dans le logement dans le corps,

fermeture du couvercle du corps,

écrasement du canal avec la proéminence sur le couvercle, formant ainsi deux surfaces planes parallèles,

émission d'un rayonnement électromagnétique au travers des surfaces planes parallèles sous la forme d'une séquence d'impulsions d'au moins deux longueurs d'ondes différentes ;

détection du rayonnement diffusé dans le sang par lesdites au moins deux longueurs d'ondes ;

calcul des valeurs des paramètres sanguins corrélant les valeurs de référence au rapport entre l'intensité du rayonnement électromagnétique détecté auxdites au moins deux longueurs d'ondes.

8. Procédé selon la revendication précédente, **caractérisé en ce que** la valeur de saturation en oxygène (% $sO_2$) est obtenue par corrélation à des courbes d'étalonnage du rapport $R_2 = I_\lambda(A)I_\lambda(B)$ entre une première intensité de rayonnement électromagnétique détectée $I_\lambda(A)$ et une deuxième intensité de rayonnement électromagnétique détectée $I_\lambda(B)$ et/ou par corrélation du rapport $R_2 = I_\lambda(A)/I_\lambda(B)$ entre une intensité de rayonnement électromagnétique détectée à des valeurs d'étalonnage approximées avec une fonction mathématique du deuxième degré.

9. Procédé selon l'une quelconque des revendications 7 ou 8, **caractérisé en ce que** la valeur d'hématocrite (% Ht) est obtenue par corrélation de courbes d'étalonnage au rapport $R_1 = I_\lambda(A)/[I_\lambda(C)+ I_\lambda(D)]$ entre les intensités de rayonnement électromagnétique détectées $I_\lambda(A)$, $I_\lambda(C)$ et $I_\lambda(D)$ et/ou par corrélation du rapport $R_1 = I_\lambda(A)/[I_\lambda(C)+ I_\lambda(D)]$ entre les intensités de rayonnement électromagnétique détectées à des valeurs d'étalonnage approximées avec une fonction mathématique du troisième degré.

10. Procédé selon l'une quelconque des revendications 7 à 9, **caractérisé en ce que** la valeur de saturation en oxygène calculée du sang (% $sO_2$) est corrigée sur la base de la valeur d'hématocrite (% Ht).

11. Procédé selon l'une quelconque des revendications 7 à 10, **caractérisé en ce que** la valeur d'hématocrite calculée (% Ht) du sang est corrigée sur la base de la valeur de saturation en oxygène (% $sO_2$).

12. Procédé selon l'une quelconque des revendications 7 à 11, comprenant les étapes suivantes :

émission d'un train d'impulsions à une intensité et une fréquence constantes ;

détection du train d'impulsions ; et

traitement du train d'impulsions détecté et

association de la présence et de la taille d'un embole avec une période temporelle dans laquelle la variation d'intensité du rayonnement détecté dépasse un certain seuil.

13. Procédé selon l'une quelconque des revendications 7 à 13, **caractérisé en ce qu'**un calcul du volume d'air accumulé à la fois en tant qu'embole unique et en tant que total de micro-emboles est réalisé et en conséquence, une alarme et/ou un arrêt de l'écoulement sanguin est généré si un seuil de risque déterminé pour le patient est dépassé.

Fig. 1

Fig. 2

Fig. 3

A - A

Fig. 4

EP 2 434 952 B1

Fig. 5

EP 2 434 952 B1

Fig. 6

Fig. 7

EP 2 434 952 B1

Fig. 8

Fig. 9

EP 2 434 952 B1

Fig. 10

(a)

(b)

(c)

Fig. 11

EP 2 434 952 B1

Fig. 12

Fig. 13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6090061 A
- US 7381195 B
- US 7381195 B2
- US 6529751 B
- US 6144444 A
- US 20090043171 A1
- WO 9427495 A1
- WO 2008136548 A

**Non-patent literature cited in the description**

- *Science,* 22 June 1951, vol. 113 (2974), 723-5